# EUROPÄISCHE PATENTSCHRIFT

(11) **EP 1 282 597 B1**
(45) Veröffentlichungstag und Bekanntmachung des Hinweises auf die Patenterteilung: **16.06.2004**
(21) Anmeldenummer: 01949296.6
(22) Anmeldetag: 27.04.2001
(51) Int. Cl.: C07C 303/02, C07D 295/08

(54) **VERFAHREN ZUR HERSTELLUNG VON w-AMINOALKANSULFONSÄUREN**
METHOD FOR THE PRODUCTION OF w-AMINOALKYLSULPHONIC ACIDS
PROCEDE DE PREPARATION D'ACIDES w-AMINOALKYL SULFONIQUES

(30) Priorität: 10.05.2000 DE 10021790
(43) Veröffentlichungstag der Anmeldung: 12.02.2003
(73) Patentinhaber: Raschig GmbH, 67061 Ludwigshafen (DE)
(72) Erfinder: SCHÄFER, Volker, 67122 Altrip (DE); KNOLL, Wolfgang, 67256 Weisenheim (DE); SCHMITT, Alexander, 67127 Rödersheim-Gronau (DE); HÜTTNER, Christoph, 68529 Ladenburg (DE)
(74) Vertreter: Wagner, Jutta, Dr.
(86) Internationale Anmeldenummer: PCT/EP2001/004754
(87) Internationale Veröffentlichungsnummer: WO 2001/085678

(56) Entgegenhaltungen:
- PATENT ABSTRACTS OF JAPAN vol. 1998, no. 09, 31. Juli 1998 (1998-07-31) & JP 10 087601 A (NAGASE KASEI KOGYO), 7. April 1998 (1998-04-07)
- J.F. KING, ET AL.: "The mechanism of hydrolysis of 2-hydroxyethanesulphonyl chloride: the intermediacy of 1,2-oxathietane 2,2-dioxide (beta-sultone)" CANADIAN JOURNAL OF CHEMISTRY., Bd. 67, Nr. 12, Dezember 1989 (1989-12), Seiten 2162-2172, XP002178432 National Research Council, Ottawa, CA ISSN: 0008-4042
- CHEMICAL ABSTRACTS, vol. 92, no. 13, 31. März 1980 (1980-03-31) Columbus, Ohio, US; abstract no. 110477t, A. ZICMANIS, ET AL.: "3-Aminopropanesulphonic acids" Seite 614; XP002178434 & LATV. PSR ZINAT. AKAD. VESTIS, KHIM. SER., Nr. 5, 1979, Seiten 605-608,
- W. SCHLIEMANN, ET AL.: "Untersuchungen zur Synthese einiger 1-(p-Fluorphenyl)-1-pyrid-2'-ylbutylamine" DIE PHARMAZIE, Bd. 35, Nr. 2, Februar 1980 (1980-02), Seiten 69-72, XP002178433 GOVI-Verlag Pharmazeutischer Verlag, Eschborn, DE ISSN: 0031-7144

## Beschreibung

Die vorliegende Erfindung betrifft ein neues Verfahren zur Herstellung von ω-Aminoalkansulfonsäuren in wässriger Lösung.

### Stand der Technik:

Aminoalkansulfonsäuren gehören zur Gruppe der Sulfonamine der allgemeinen Formel I 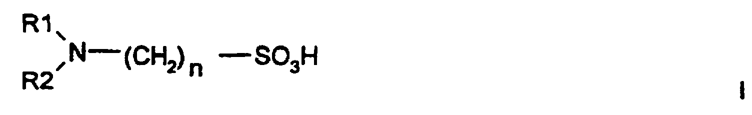 wobei R1 und R2 Alkylgruppen, und n eine ganze Zahl von 1 bis 5 sind.

Die Herstellung einer Gruppe in dieser Verbindungen mit n = 3 erfolgt gemäß dem Stand der Technik mittels 1,3 - Propansulton nach der allgemeinen Reaktionsgleichung

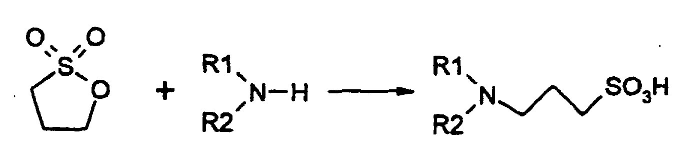

Das für dieses Verfahren eingesetzte 1,3 - Propansulton reagiert hierbei als hoch reaktives Agens auch mit schwachen Nucleophilen. In der hohen Reaktivität liegt aber auch ein hohes physiologisches Potential begründet (Chem.Abs. 1980, 92(13), 110477t)

Alkansultone sind daher als potentiell krebserzeugend eingestuft und die Verwendung aus diesem Grunde nachteilig. Die Ausbeute dieser Reaktion liegt jedoch allgemein bei 95 bis 98 Prozent, so daß sie bisher die technisch wichtigste Herstellungsweise darstellt.

Beispiele für technisch verwendete Alkansulfonsäuren sind:

2-Morpholinethansulfonsäure und 4-(2-Hydroxyethyl)-1-piperazinpropansulfonsäure. Diese Verbindungen dienen u.a. als biologische Puffersubstanzen in Zellkulturen.

Die EP 0 752 420 A1 beschreibt ein Verfahren zur Herstellung von 3-Pyridiniumpropansulfonbetain (PPS) unter Umgehung von 1,3 - Propansulton wobei Pyridin mit 1,3-Dihalogenpropan und Natriumsulfit in Gegenwart von Wasser und einem Alkylhalogenid als Lösungsmittel umgesetzt wird.

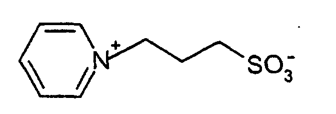

Das Verfahren ist jedoch nur für tertiäre Aminkomponenten geeignet, die keinen Wasserstoff am Aminstickstoff tragen. Bei Einsatz von sekundären Aminen erfolgt nämlich eine Deprotonierung des gebildeten tertiären Ammoniumsalzes durch noch nicht umgesetzte sekundäre Amine, da das sekundäre Amin eine stärkere Base darstellt als ein tertiäres Amin und der entstehende quartemäre Stickstoff ein leicht abspaltendes Wasserstoff trägt. Dadurch freigesetztes tertiäres Amin kann nun erneut mit dem Dihalogenpropan reagieren und damit unerwünschte quartemäre Produkte im Sinne der folgenden Reaktionsgleichung bilden.

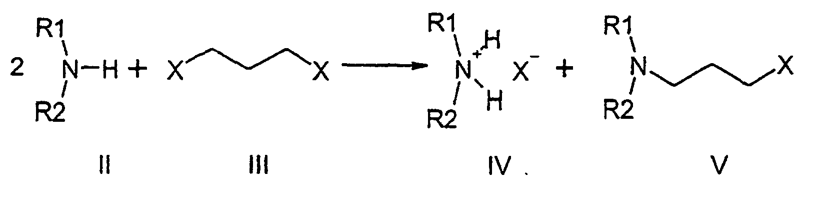

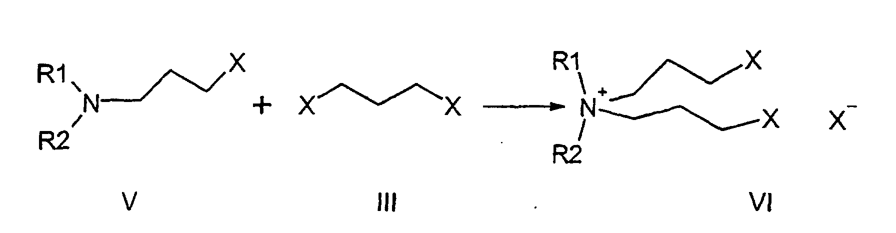

Dies führt neben der erwünschten Zwischenverbindung V zu dem nicht mehr weiterreagierenden Ammoniumhalogenidsalz IV und dem unerwünschten quartären Salz VI. Dies führt zu erheblichen Ausbeuteminderungen und hohen Anteilen an Nebenprodukten.

Weiter ergibt sich eine hohe Belastung der erhaltenen Lösungen mit Alkalihalogeniden. Diese ist jedoch bei den geforderten hohen Reinheiten der Endprodukte, beispielsweise als Biopuffer nicht vertretbar, so daß nach den bekannten Herstellungsverfahren eine Isolierung und Reinigung der festen Aminoalkansultonsäuren erfolgen muß.

Somit steht bisher kein Zugang zu sulfoalkylierten, beispielsweise sulfopropylierten sekundären Aminverbindungen unter Ausschluß von Alkansulfonen offen.

Es stellte sich daher die Aufgabe, ein Verfahren zu finden, mit dem auch ω-Aminoalkansulfonsäuren ohne Alkansultone wirtschaftlich hergestellt werden können und das vorzugsweise auch eine Aufreinigung der erhaltenen Lösungen ohne Isolierung der Festprodukte gestattet.

Diese Aufgabe wird gemäß den Merkmalen des Hauptanspruchs gelöst und durch die Merkmale der Unteransprüche gefördert.

Die Erfindung betrifft demnach ein Verfahren zur Herstellung von Aminoalkansulfonsäuren, vorzugsweise als wässrige Lösung, ohne den Einsatz von Alkansultonen. Weiterhin die Reinigung der erhaltenen Lösungen und die zum Erhalt der reinen Substanzen notwendigen Schritte. Weiteres Ziel des neuen Verfahrens ist es; ebenso hohe Ausbeuten wie bei den Alkansulton-Verfahren bei gleicher Produktqualität zu erhalten. Daher müssen mögliche Nebenreaktionen unterdrückt, sowie eine möglichst vollständige Reaktion der eingesetzten Stoffe erreicht werden.

Da es nicht möglich ist, sekundäre Amine direkt mit Dihalogenalkanen und Alkalisulfit nebenproduktfrei zur Reaktion zu bringen, ist es notwendig, eine neues Verfahren zur Herstellung anzuwenden.
Hierbei wird von sekundären Aminen ausgegangen, welche mit Dihalogenalkanen dergestalt zur Reaktion gebracht werden, daß der Zusatz von Alkalilauge erfolgt. Dies ist notwendig, um etwaige Nebenreaktionen zu unterbinden. Es wird in wasserhaltigen Medien gearbeitet.

In einem zweiten Schritt wird die Zugabe von Halogenwasserstoffsäure zur Reaktionslösung notwendig, um einen für die weiteren Schritte des Herstellprozesses günstigen sauren pH-Bereich zu erreichen.

Zur Bildung der Sulfonsäure wird der Reaktionslösung Alkalisulfit in zum Amin äquimolaren Mengen zugesetzt.

Störend für den direkten Einsatz der erhaltenen Produkte, beispielsweise der Puffersubstanz, wirken sich die erhaltenen Mengen an Alkalihalogeniden, sowie die in Spuren entstehenden Nebenprodukte aus. Um das Produkt in der für Biopuffer-Lösungen geforderten Reinheit zu erhalten, muß nach der Reaktion eine Aufreinigung des wässrigen Reaktionsmediums durchgeführt werden. Es ist hierbei zu berücksichtigen, daß pro 1 Mol Reaktionsprodukt mindestens 2 Mol Alkalihalogenide entstehen. Aufgrund eines geringen Überschusses an Alkalisulfit im Reaktionsansatz ist der Anteil an Alkalisalzen in der Produktlösung jedoch zusätzlich noch um diesen Anteil erhöht. Eine Abreicherung der Alkalihalogenide und Nebenprodukte kann nun beispielsweise mittels lonenaustauschverfahren durchgeführt werden. Dies ist jedoch aufgrund des hohen Gehaltes an gebildeten Alkalihalogeniden nicht wirtschaftlich durchführbar. Das Nanofiltrationsverfahren alleine reicht aus technischen Gründen ebenfalls nicht aus, die geforderte Produktreinheit zu erreichen, da in der Nanofiltration alle negativ zweiwertigen geladenen Teilchen und größeren Moleküle, wie sie als Nebenprodukte entstehen, ebenfalls zurückgehalten werden und so in der Produktlösung verbleiben.

Die in der vorliegenden Erfindung dargestellte Produktreinigung bezieht sich nun auf die Kombination aus Nanofiltration und lonenaustauschverfahren zur wirtschaftlichen Abtrennung des Produktes von den während der Reaktion entstehenden ein - und zweiwertigen Alkalihalogeniden und Nebenprodukten. Die im Rahmen dieser Erfindung eingesetzten Verfahren der Nanofiltration und des lonenaustausches mittels lonenaustauscherharzen sind bekannt.

Die Nanofiltration dient hierbei zur Abreicherung der einwertigen Alkylhalogenide. Die zweiwertigen Alkalihalogenide und restlichen Nebenprodukte, die einen gemeinsamen Anteil von ca. 0,5 Gew.-% ausmachen, werden in einem zweiten Schritt mittels lonenaustauschverfahren entfernt. Die Durchführung des erfindungsgemäßen Verfahrens ist nachfolgend in allgemeiner Form beschrieben.

### Die Nanofiltration

Der osmotische Druck der Reaktionslösung, welcher während der Nanofiltration zur Abreicherung der einwertigen Alkalihalogenide überwunden werden muß, wird durch die Konzentration des Produktes, der Nebenprodukte und der zweiwertigen Alkalisalze bestimmt. Dieser osmotische Druck beträgt in der üblichen Reaktionslösung ca. 30 Bar. Zur Durchführung der Nanofiltration bei technisch und wirtschaftlich sinnvollen Betriebsdrücken muß die Reaktionslösung daher in einem Verhältnis von 1 : 2 bis 1 : 3 mit Wasser verdünnt werden, wobei sich der osmotische Druck in gleicher Weise auf 15-10 Bar reduziert. Das Nanofiltrationsverfahren wird vorzugsweise in der Verfahrensweise der Diafiltration durchgeführt. Für eine ausreichende Abreicherung der Alkalihalogenide wird VE-Wasser als Diafiltrationslösung verwendet. Das Diafiltratvolumen entspricht etwa dem 7 - 10fachen der Reaktionslösung. Damit ist eine Abreicherung der einwertigen Alkalihalogenide > 95 % möglich. Eingesetzt wird eine Nanofiltrationsmembran mit einem Cutt-Off von ca. 150 - 300 g/Mol, vorzugsweise ca. 200 g/Mol und ein Betriebsdruck von 25 - 35 Bar, d.h. 15 - 20 Bar höher als der osmotische Druck eingestellt. Im Anschluß an die Diafiltration wird die jetzt teilweise aufgereinigte Reaktionslösung auf das Ursprungsvolumen aufkonzentriert und durch lonenaustauschverfahren weiter aufgereinigt.

### lonenaustauschverfahren

Der Austausch von Anionen und Kationen mittels lonenaustauscherharzen wird seit langem benutzt und ist Stand der Technik. Die hier beschriebene Aufreinigung wird mit einer Kombination aus einem stark sauren Kationenaustausch und einem schwach basischen Anionenaustausch durchgeführt. Eingesetzt wurde beispielsweise als stark saurer ionenaustauscher ein Typ Pyrolite C 104 und als schwach basischer lonenaustauscher ein Typ Pyrolite A 100. Die Betriebsweise der lonenaustauscher soll in einem Gleich- oder Gegenstromverfahren durchgeführt werden, das Gleichstromprinzip wird bevorzugt. Das Verhältnis von Anionen und Kationenaustauscher wird so eingestellt, daß der nachfolgende Anionenaustauscher die gleiche Austauscherkapazität wie der Kationenaustauscher aufweist. Die Kapazität der lonenaustauscher reicht aus, um ein durch die Nanofiltration vorgereinigtes Reaktionsvolumen entsprechend dem 8 - 10fachen Bettvolumen des Kationenaustauschers abzureichem. Das lonenaustauschverfahren ermöglicht eine Abreicherung der Alkalihalogenide und Nebenprodukte auf weniger als 0,1 %. Zur Elution des im lonenaustauscher verbleibenden Produktes werden die lonenaustauschersäulen mit VE-Wasser gewaschen. Das Waschwasservolumen entspricht etwa dem 2 - 3fachen Bettvolumen des Kationenaustauschers. Die Regeneration der lonenaustauscher findet entsprechend den technischen Angaben der Hersteller statt.

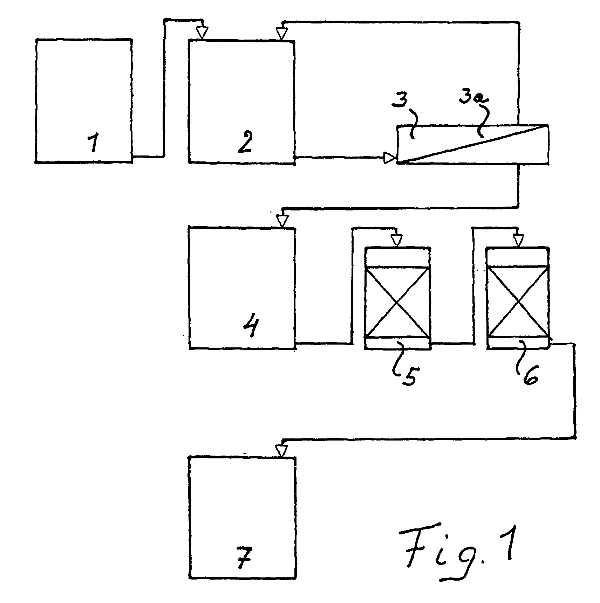

In der beigefügten Fig. 1 wird das Verfahrensfließbild der erfindungsgemäßen Kombination von Nanofiltration und lonenaustausch zur Produktreinigung wiedergegeben. In dieser Figur wird das Diafiltrat aus Behälter 1 in den Produktbehälter 2 überführt, von dem es in die Nanofiltration 3 läuft, in welcher durch die diagonale Linie die Nanofiltrationsmembran 3a angedeutet ist. Die die Membran nicht passierenden Anteile werden in die Produktlösung zurückgeführt und dort mit Diafiltrat in die Reinigungskonzentration zurückverdünnt. Die durch die Membran 3a laufende gereinigte Lösung geht über einen Zwischentank 4 auf die erste lonenaustauschersäule mit einem stark kationischen Austauscher 5 und deren Ablauf auf eine zweite lonenaustauschersäule mit schwach anionischem Austauscher 6. Das durch diese lonenaustauscher gereinigte Produkt wird in einem Auffangtank 7 gesammelt und nach Bedarf entnommen.

### Verfahrensbeispiel:

### Herstellung von 3-Morpholinopropansulfonsäurelösung ω-(MOPS)

- 6298 g (40 Mol) an 1,3-Bromchlorpropan (BCP) wurde vorgelegt und auf 20°C temperiert, 420 g (10 Mol) NaOH in 2000 ml Wasser wurde zugegeben und anschließend 870 g (10 Mol) Morpholin zugegeben.
- Nach 10 min wurde die Suspension auf 45°C aufgeheizt. Bei dieser Temperatur wurde 8 Stunden gerührt.
- 100 ml 33 %ige Salzsäure in 1250 g Wasser im leichten Überschuß (ca. 11 Mol) wurde zugegeben. Dabei ist nicht die Menge an Salzsäure zu beachten, sondern der pH-Wert. Erst wenn dieser auf 0 bis 1 abgesunken ist, tritt eine scharfe Trennung zwischen wässriger und organischer Phase auf.
- Die organische Phase (Überschuß BCP) wurde abgelassen.
- Zur wässrigen Phase wird konzentrierte Natronlauge bis zu einem pH-Wert von 5 - 6 und anschließend eine gesättigte Natriumsulfitlösung (1260 g (10 Mol) NaSO₃ + 1250 g H₂O) zugefügt.
- Die Mischung wurde auf 75°C aufgeheizt und bei dieser Temperatur 24 Stunden gerührt.
- Danach wurde die Lösung abgekühlt und wie folgt aufgereinigt.

### Aufreinigung mittels Nanofiltration und lonenaustauscher

Eingesetzt wurden 7,8 kg ( 6,5 Liter) der salzhaltigen Produktlösung aus der Herstellung der ω-MOPS. Die Produktlösung enthielt 1,21 kg ω-MOPS, 0,73 kg NaCl, 0,69 kg NaBr und 0,04 kg Na₂SO₄. Die Abtrennung der einwertigen Salze NaCl und NaBr wurde mittels Nanofiltration durchgeführt. Zur Reduktion des osmotischen Druckes der Lösung wurde die Produktlösung mit 6,5 Liter VE-Wasser verdünnt. Die Nanofiltration wurde in Form einer Diafiltration mit einem konventionellen Wickelmodul durchgeführt. Eingesetzt wurde ein Wickelmodul der Fa. Osmonics mit einem Cutt-Off von 250 Dalton und einer Membranfläche von 1 m². Die Nanofiltration fand bei einem Transmembrandruck von 35 bar und einer Überströmung der Membran von 1000 l/m²h statt.
Die Nanofiltration wurde wie folgt durchgeführt: Die salzhaltige Produktlösung wurde im Vorlagebehälter der Nanofiltrationsanlage vorgelegt und mit 6,5 Liter VE-Wasser verdünnt. Das abfließende Permeatvolumen (mittlerer Permeatfluß 50 1/h) wurde kontinuierlich im gleichen Verhältnis im Vorlagebehälter durch VE-Wasser ersetzt (Diafiltration). Die Nanofiltration wurde solange durchgeführt, bis ein Permeatvolumen von 100 Liter aus dem System ausgeschleust war. Das Permeat wurde in einem Tank aufgefangen. Danach wurde eine AufKonzentrierung der jetzt weitgehend entsalzten Produktlösung durchgeführt, bis das Ursprungsvolumen von 6,5 Liter wieder erreicht war. Nach der Diafiltration des Reaktionsmediums wurde eine Aufkonzentrierung der 100 Liter Permeat bis auf ein Endvolumen von 7,5 Liter durchgeführt. Die Einstellung waren hier eine Membranüberströmung von 1000 l/m²h und ein Transmembrandruck von 30 bar. Das aufkonzentrierte Permeat und die teilentsalzte Produktlösung wurden danach miteinander gemischt (nachfolgend vorgereingte Produktlösung genannt). Die Permeataufkonzentrierung hatte den Zweck, ca. 90% der MOPS aus dem Permeat wieder zurückzugewinnen und damit den produktbezogenen Gesamtrückhalt in der Nanofiltration von 90% auf über 99% zu steigern.
Nach der Nanofiltration enthielt die Mischung aus teilentsalzter Produktlösung und aufkonzentriertem Permeat 1,20 kg ω-MOPS, 0,045 kg NaCl, 0,037 kg NaBr und 0,04 kg Na₂SO₄. Die Nanofiltration ermöglicht somit eine Reduktion von NaCl um 94% und von NaBr um 95% bei einem Produktverlust kleiner 1%, der Gehalt an Natriumsulfat wird nicht reduziert.

Im Anschluß an die Nanofiltration fand die Abtrennung des Natriumsulfates und der restlichen Salze aus der vorgereingten Produktlösung mittels lonenaustauschverfahren statt. Ein Liter der vorgereinigten Produktlösung enthielt 0,144 kg ω-MOPS, 2,8 g NaCl, 3,5g NaBr und 3g Na₂SO₄, Eingesetzt wurde hier eine Anordnung aus zwei Säulen mit 350 ml des stark kationischen lonentauschers Relite EXC08 und 170 ml des schwach anionischen lonentauschers Relite EXA54. Die Beladungskapazität des lonentauschermaterials reicht aus, um 1 Liter der vorgereinigten Produktlösung vollständig zu entsalzen. Die lonentauscher werden in ihrer entsprechend regenerierten Form (H-Form für stark kationisch und OH-Form für schwach anionisch) eingesetzt. Die vorgereinigte Produktlösung wurde zuerst über den stark kationischen lonentauscher und danach über den schwach anionischen lonentauscher geleitet. Die Durchströmung der vorgereinigten Produktlösung durch die lonentauscher erfolgte durch den hydrostatischen Druck. Danach wurden beide lonentauscher mit 1 Liter VE-Wasser gespült. Das Spülwasser und die jetzt vollständig aufgereinigte Produktlösung werden miteinander gemischt und dieses Gemisch ergab 2 Liter Produktlösung. Die Produktlösung enthielt nach der Aufreinigung mittels lonenaustauschverfahren 0,138 kg ω-MOPS und keine nachweisbaren Salze mehr. Dies entsprach einem Produktverlust kleiner 4%. Hiermit war die Aufreinigung beendet.

## Patentansprüche

1. Verfahren zur Herstellung von ω-Aminoalkylsulfonsäuren der allgemeinen Formel I 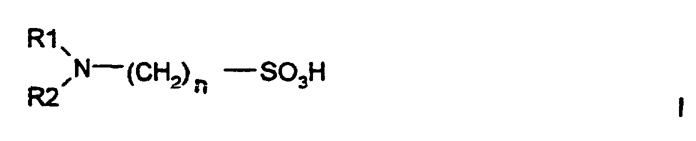 in der R1 und R2 gleich oder verschieden sind und geradkettige oder verzweigte Alkylgruppen mit 1 - 20 C-Atomen darstellen oder zusammen mit dem Stickstoffatom einen 5- oder 6-gliedrigen gesättigten aliphatischen Ring bilden, dabei 1 oder 2 Glieder des Ringe statt CH₂ auch N-R3,O oder S sein können, R3 Wasserstoff oder eine Alkylgruppe mit 1 - 20 C-Atomen darstellt und
n eine ganze Zahl von 2-6 darstellt, **dadurch gekennzeichnet, daß** man ein Amin der Formel II 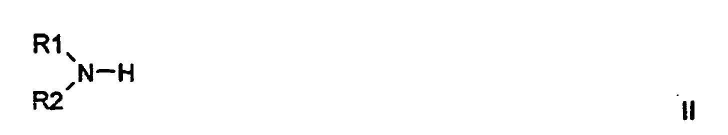 in der R1 und R2 die obige Bedeutung haben, mit einem Alkyldihalogenid der Formel III 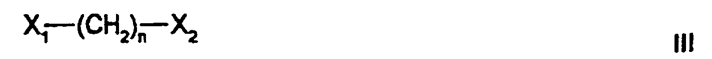 in der n die obige Bedeutung hat, X₁ und X₂ = Chlor oder Brom sind und Alkalisulfit in wässriger Lösung umsetzt, wobei zunächst Amin und Alkyldihalogenid unter Zugabe von Alkalihydroxid bei einem pH-Wert von 8-10 zur Reaktion gebracht werden und anschließend durch Zugabe von Halogenwasserstoffsäure ein pH-Wert von 0-1 eingestellt wird und überschüssiges Alkyldihalogenid abgetrennt wird, bevor die Reaktionslösung mit Alkalilauge auf einen pH-Wert von 6-7,5 eingestellt wird, Alkalisulfit zugefügt wird und bei erhöhter Temperatur das Produkt der Formel 1 gebildet wird.

2. Verfahren nach Anspruch 1, **dadurch gekennzeichnet, daß** als Alkali Natrium und als Halogen Chlorid eingesetzt wird.

3. Verfahren nach Anspruch 1 oder 2, **dadurch gekennzeichnet, daß** die Reaktionslösung durch Nanofiltration von enthaltenen Alkalihalogeniden und -sulfiden getrennt wird.

4. Verfahren nach Anspruch 3, **dadurch gekennzeichnet, daß** die Reaktionslösung zur Nanofiltration auf eine Konzentration mit einem osmotischen Druck von 10 - 20 bar eingestellt wird.

5. Verfahren nach Anspruch 3 oder 4, **dadurch gekennzeichnet, daß** an die Nanofiltration eine Reinigung mittels lonenaustauscherchromatographie angeschlossen wird, wobei mittels einem stark sauren Kationenaustauscher und einem schwach basischen Anionenaustauscher die Bisulfide und nicht amphoteren Ausgangs- und Nebenprodukte abtrennt.

## Claims

1. Process for the preparation of ω-aminoalkylsulphonic acids of the general formula I 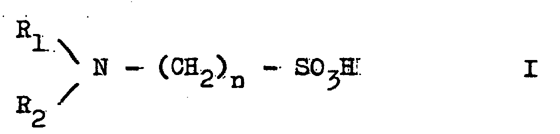 in which R₁ and R₂ are the same or different and represent straight-chained or branched alkyl groups with 1 - 20 C-atoms or, together with the nitrogen atom, form a 5- or 6-membered saturated aliphatic ring, 1 or 2 members of the ring can thereby also be N-R₃, O or S instead of CH₂, R₃ represents hydrogen or an alkyl group with 1-20 C-atoms, and n represents a whole number from 2-6, **characterised in that** one reacts an amine of the formula II 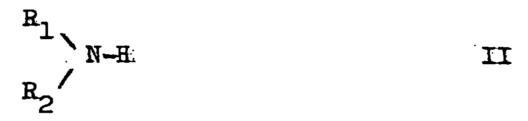 in which R₁ and R₂ have the above meaning, with an alkyl dihalide of the formula III
X₁ - (CH₂)ₙ - X₂ III
in which n has the above meaning, X₁ and X₂ = chlorine or bromine, and alkali sulphite in aqueous solution, whereby first amine and alkyl dihalide are brought to reaction with addition of alkali. hydroxide at a pH value of 8 - 10 and subsequently, by addition of hydrohalic acid, a pH value of 0 - 1 is adjusted and excess alkyl dihalide is separated off before the reaction solution is adjusted with alkali lye to a pH value of 6 - 7.5, alkali sulphite is added thereto and the product of formula I is formed at elevated temperature.

2. Process according to claim 1, **characterised in that** sodium is used as alkali and chloride as halogen,

3. Process according to claim 1 or 2, **characterised in that** the reaction solution is separated by nanofiltration from alkali halides and sulphides contained.

4. Process according to claim 3, **characterised in that** the reaction solution is adjusted for the nanofiltration to a concentration with an osmotic pressure of 10 - 20 bar.

5. Process according to claim 3 or 4, **characterised in that** after the nanofiltration there follows a purification by means of ion exchanger chromatography, whereby, by means of a strongly acidic cation exchanger and a weekly basic anion exchanger, the bisulphides and non-amphoteric starting and by-products are separated off.

## Revendications

1. Procédé de préparation d'acides ω-aminoalkylsulfoniques de formule générale I 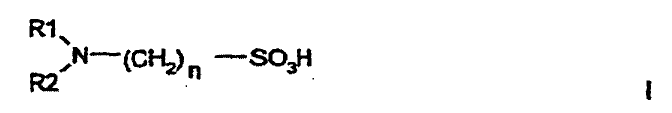 dans laquelle R1 et R2 sont identiques ou différents et représentent des groupes alkyle à chaîne droite ou ramifiée comprenant de 1 à 20 atomes de carbone ou forment ensemble avec l'atome d'azote un noyau aliphatique saturé à 5 ou 6 chaînons, un ou deux chaînons du noyau pouvant également représenter, outre CH₂, N-R3, O ou S, R3 représente un hydrogène ou un groupe alkyle comportant de 1 à 20 atomes de carbone et
n représente un nombre entier allant de 2 à 6,
**caractérisé en ce qu'**on fait réagir une amine de formule II 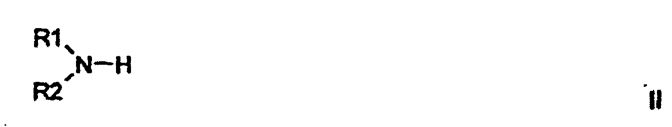 dans laquelle R1 et R2 ont la signification donnée ci-dessus, avec un dihalogénure d'alkyle de formule III
X₁-(CH₂)ₙ-X₂ III
dans laquelle n a la signification donnée ci-dessus, X₁ et X₂ représentent un chlore ou un brome et un sulfite de métal alcalin en solution aqueuse,
en mettant tout d'abord à réagir l'amine et le dihalogénure d'alkyle en ajoutant un hydroxyde de métal alcalin à un pH de 8 à 10, puis en établissant, par l'ajout d'un acide halogénhydrique, une valeur de pH allant de 0 à 1 et en séparant l'excès de dihalogénure d'alkyle, avant de régler la solution réactionnelle, avec une base alcaline, à une valeur de pH allant de 6 à 7,5, en ajoutant un sulfite de métal alcalin et en formant à température élevée le produit de formule 1.

2. Procédé selon la revendication 1, **caractérisé en ce qu'**on utilise comme métal alcalin le sodium et comme halogène le chlorure.

3. Procédé selon la revendication 1 ou 2, **caractérisé en ce qu'**on sépare la solution réactionnelle, au moyen d'une nanofiltration, des halogénures et sulfures de métaux alcalins qui y sont contenus.

4. Procédé selon la revendication 3, **caractérisé en ce qu'**on règle la solution réactionnelle, en vue de la nanofiltration, à une concentration présentant une pression osmotique de 10 à 20 bars.

5. Procédé selon la revendication 3 ou 4, **caractérisé en ce qu'**on adjoint à la nanofiltration une purification au moyen d'une chromatographie d'échange d'ions, dans laquelle, au moyen d'un échangeur de cations fortement acide et d'un échangeur d'anions faiblement basique, on sépare les bisulfures et les produits de départ et sous-produits non amphotères.
